# EUROPEAN PATENT APPLICATION

(11) **EP 2 990 117 A1**
(43) Date of publication of application: **02.03.2016**
(21) Application number: 14188147.4
(22) Date of filing: 08.10.2014
(51) Int. Cl.: B01L 3/00, G01N 1/10, G01N 33/483, G01N 35/10

(54) **Equal-liquid-level reservoir and a microfluidic biochip**

(30) Priority: 19.08.2014 CN 201410408919
(71) Applicant: Shenzhen Createcare Medical Instrument Co., Ltd., Shenzhen City, Guangdong (CN)
(72) Inventor: Zhuang, Bin, Shenzhen City Guangdong (CN)
(74) Representative: Karakatsanis, Georgios

(57) **Abstract**

A microfluidic biochip includes an equal-liquid-level reservoir disposed on a cover. The equal-liquid-level reservoir includes some tanks that have a substantially same liquid level. Each tank has an opening on a bottom surface, each opening communicating with a corresponding microfluidic channel.

## Description

### BACKGROUND OF THE INVENTION

### 1. FIELD OF THE INVENTION

The present invention generally relates to a biochip, and more particularly to a microfluidic biochip with an equal-liquid-level reservoir.

### 2. DESCRIPTION OF RELATED ART

Microfluidics is a multi-disciplinary technology intersecting engineering, physics, chemistry, biochemistry, nanotechnology and biotechnology. Microfluidics may be applied to separation or detection by manipulating small volumes of fluids with advantages of small size and low power consumption. Microfluidics may be utilized to manufacture biochips with applications, for example, to detecting motility or quality of sperms.

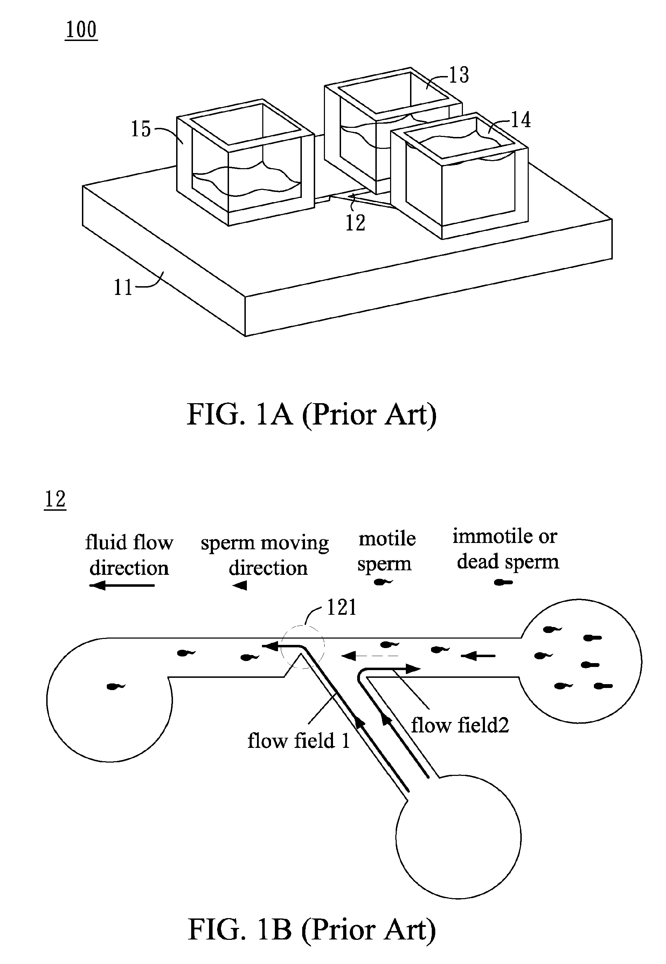

FIG. 1A shows a perspective view of a conventional biochip 100. Microfluidic channels 12 are formed in a substrate 11, and reservoirs 13, 14 and 15 are respectively disposed above and connected to openings of the microfluidic channels 12, where the reservoir 13 may store a sperm specimen. FIG. 1B shows a top view of the microfluidic channels 12. The fluid velocity of the flow field 2 in the microfluidic channels 12 may be controlled by liquid-level difference between the reservoirs 13 and 14. As sperm cells normally have a moving velocity ranging between 50 and 70 micrometers per second, the fluid velocity in the microfluidic channels 12 may range between 0 and 50 micrometers per second. When the fluid velocity in the microfluidic channels 12 is greater than 0, the sperm cells move upstream; when the fluid velocity in the microfluidic channels 12 is substantially equal to 0, the sperm cells move freely in the microfluidic channels 12. In both cases, collected at a micro-pore 121 of the microfluidic channels 12 are motile sperm cells instead of immotile or dead sperm cells. Accordingly, the motile cells in the sperm specimen may be counted statistically via collection and statistical algorithm. As the liquid-level difference between the reservoirs 13 and 14 is usually small, a normal user has difficulty precisely adding the required amount of fluid to arrive at a controlled fluid velocity, thereby resulting in instability of the liquid-level difference between the reservoirs 13 and 14, and greatly reducing the accuracy of biological detection. The fluid velocity of the flow field 1 in the microfluidic channels 12 may be controlled by liquid-level difference between the reservoirs 14 and 15. The fluid velocity of the flow field 1 is greater than that of the flow field 2, and is used to flush the sperm cells through the micro-pore 121. The fluid velocity of the flow field 1 is related to frequencies of generated pulse signals. As the compatibility with the frequencies of generated pulse signals is ordinarily high and the accuracy requirement of this fluid velocity is low, it is acceptable that the liquid-level of the reservoir 14 is higher than liquid-level of the reservoir 15 with at least 10 millimeters.

A need has thus arisen to propose a novel biochip for improving instability of the liquid-level difference in the conventional biochip.

### SUMMARY OF THE INVENTION

In view of the foregoing, it is an object of the embodiment of the present invention to provide a biochip with an equal-liquid-level reservoir to alleviate instability of liquid-level difference in the conventional reservoir, and to improve accuracy of biological detection.

According to one embodiment, the microfluidic biochip includes a substrate, a cover and an equal-liquid-level reservoir. Microfluidic channels are formed in the substrate, the cover is disposed above the substrate, and the equal-liquid-level reservoir is disposed on the cover. The equal-liquid-level reservoir includes plural tanks that have a substantially same liquid level. Each tank has an opening on a bottom surface, and each opening communicates with a corresponding microfluidic channel.

### BRIEF DESCRIPTION OF THE DRAWINGS

FIG. 1A shows a perspective view of a conventional biochip;
FIG. 1B shows a top view of the microfluidic channels of FIG. 1A;
FIG. 2A shows a top view of a biochip according to one embodiment of the present invention;
FIG. 2B shows a top view of one microfluidic assembly of FIG. 2A;
FIG. 2C shows an equivalent circuit representing the biochip of FIG. 2A;
FIG. 3 shows a top view of a biochip according to another embodiment of the present invention;
FIG. 4A shows a perspective view of a biochip according to one embodiment of the present invention;
FIG. 4B shows a top view of a biochip according to one embodiment of the present invention;
FIG. 4C shows a cross-sectional view of the equal-liquid-level reservoir of FIG. 4A; and
FIG. 4D shows another cross-sectional view of the equal-liquid-level reservoir of FIG. 4A.

### DETAILED DESCRIPTION OF THE INVENTION

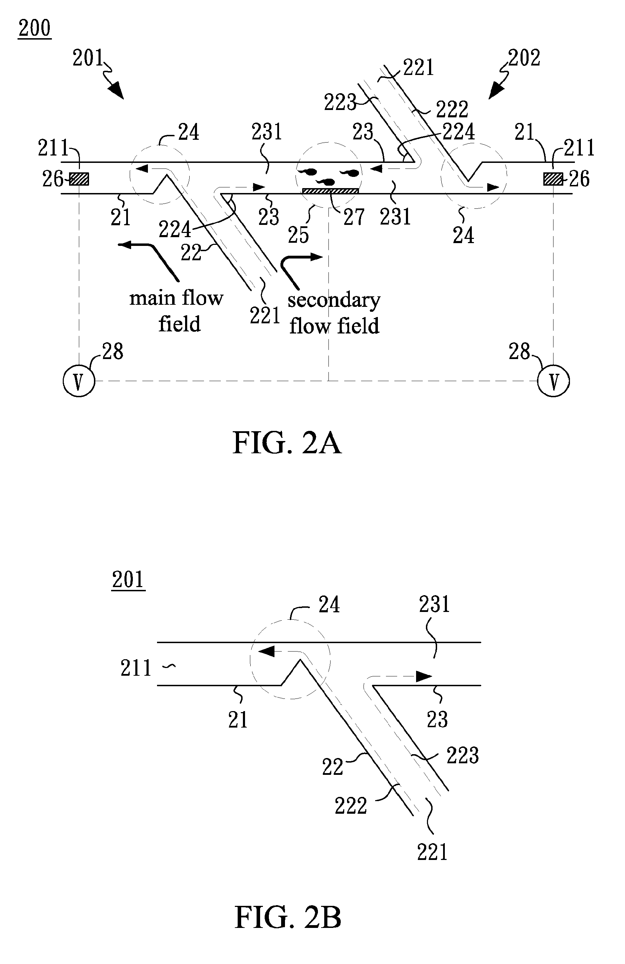

FIG. 2A shows a top view of a biochip 200 according to one embodiment of the present invention. In the embodiment, the biochip 200 may be used to detect motility or quality of sperms. The invention may generally be used to detect motility of other single-cellular or multi-cellular organisms, which are referred as biological specimens.

The biochip 200 of the embodiment includes plural microfluidic assemblies 201 and 202 formed in a substrate such as glass. FIG. 2B shows a top view of one microfluidic assembly (e.g., 201) of FIG. 2A. The microfluidic assemblies of the microchip 200 may be the same or may be different for specific purpose. As illustrated in FIG. 2B, the microfluidic assembly 201 includes a first microfluidic channel 21, a second microfluidic channel 22 and a third microfluidic channel 23. First ends of the first microfluidic channel 21, the second microfluidic channel 22 and the third microfluidic channel 23 are connected at a junction 24. An opening at the second end 211 of the first microfluidic channel 21 is connected to a reservoir; an opening at the second end 221 of the second microfluidic channel 22 is connected to another reservoir; and an opening at the second end 231 of the third microfluidic channel 23 is connected to another microfluidic assembly 202 at a joint 25, as shown in FIG. 2A.

According to one aspect of the embodiment, the cross-sectional area of the junction 24 is substantially less than the cross-sectional areas of the first microfluidic channel 21, the second microfluidic channel 22 and the third microfluidic channel 23, thereby enhancing sensitivity of biological detection. In the embodiment, the first microfluidic channel 21, the second microfluidic channel 22 and the third microfluidic channel 23 may have 10-10000 micrometers in width, and 5-500 micrometers in depth. The junction 24 may have 5-100 micrometers in width, and may have depth the same as or less than the first microfluidic channel 21, the second microfluidic channel 22 and the third microfluidic channel 23. Scheme of making the depth of the junction 24 less than other microfluidic channels may be referenced to China patent No. 103398924, entitled "IMPROVED BIOCHIP MICRO-POROUS SENSOR" by the same inventor of the present application, disclosure of which is incorporated herein by reference.

The first microfluidic channel 21 and the third microfluidic channel 23 of the embodiment are arranged in a substantially straight line, and an intersection angle 224 (e.g., an acute angle) is defined between the second microfluidic channel 22 and the third microfluidic channel 23. Accordingly, the fluid velocity 222 from the second microfluidic channel 22 toward the first microfluidic channel 21 may be different from the fluid velocity 223 from the second microfluidic channel 22 toward the third microfluidic channel 23.

Still referring to FIG. 2A, a pair of electrodes 26 and 27 is disposed in a corresponding microfluidic assembly (e.g., 201). For example, the electrodes 26 and 27 are disposed at the second end 211 of the first microfluidic channel 21 and the second end 231 of the third microfluidic channel 23 respectively, and a detector 28 is electrically connected between the electrodes 26 and 27. The electrode 27 mentioned above may be a common electrode between the microfluidic assemblies 201 and 202.

According to the architecture of the biochip 200 of FIG. 2A, when a biological specimen (e.g., sperms) enters the opening at the second end 221 of the second microfluidic channel 22 of the (left) microfluidic assembly 201, a portion of the sperms may pass the junction 24 of the (left) microfluidic assembly 201, and may be detected by the associated electrodes 26, 27 and the detector 28 with voltage pulses; and the other portion of the sperms may pass the junction 24 of the (right) microfluidic assembly 202, and may be detected by the associated electrodes 26, 27 and the detector 28 with voltage pulses. In the embodiment, at least one pair of electrodes 26 and 27 is used, and an associated detector 28 is used to detect voltage pulses.

When the reservoirs at the second ends 221 (of the second microfluidic channel 22) of the two microfluidic assemblies have substantially equal liquid-level, liquid pressures at the second ends 231 (of the third microfluidic channel 23) are thus substantially the same, and therefore the liquid between the second ends 231 approximately reaches standstill with fluid velocity of substantial zero. In this situation, sperm cells reaching the junction 24 of the microfluidic assembly 202 are motile sperms that move themselves. Sperm cells reaching the junction 24 of the microfluidic assembly 201 include motile sperms and immotile sperms, that is, total sperms, flushed by the main flow field.

According to the embodiment described above, different intersection angle 224 between the second microfluidic channel 22 and the third microfluidic channel 23 may be adopted. Generally speaking, larger intersection angle 224 results in greater liquid pressure in the secondary flow field. Different cross-sectional areas of the first microfluidic channel 21, the second microfluidic channel 22 and the third microfluidic channel 23 may be adopted, for example, by adjusting width and depth. Generally speaking, larger cross-sectional area results in smaller passage resistance and greater liquid pressure. Longer microfluidic channel results in larger passage resistance and lesser liquid pressure. Different cross-sectional area at the junction 24 may be adopted, for example, by adjusting width and depth. Generally speaking, larger cross-sectional area results in larger liquid flow.

By implementing different secondary flow field (i.e., different fluid velocity and/or flow direction), liquid at the joint 25 between the second ends 231 of the third microfluidic channels 23 may be made forward flow, backward flow or standstill. The fluid velocity of each microfluidic channel may be fine tuned according to requirement or purpose of the biological detection function of a biochip.

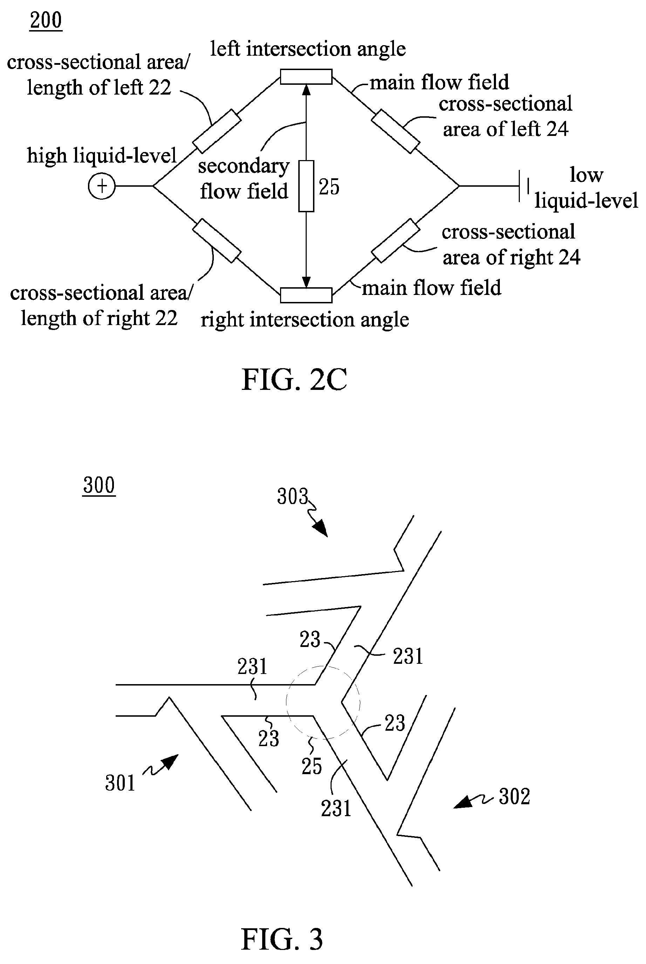

The system of the biochip 200 may be represented and interpreted by an equivalent circuit shown in FIG. 2C. Liquid pressure is equivalent to voltage, liquid velocity is equivalent to current, cross-sectional area and length are equivalent to resistance, and intersection angle is equivalent to adjustable resistance. It is noted that the system of the biochip 200 may be equivalent to an electric bridge.

Some exemplary embodiments are described in the following. In a first exemplary embodiment, same liquid (or liquid with same viscosity) is adopted in the left and right microfluidic assemblies 201 and 202. Liquid at the joint 25 approximately reaches standstill because the liquid pressures at both sides of the joint 25 are substantially the same, provided that the two microfluidic assemblies 201 and 202 are symmetrical to each other.

In a second exemplary embodiment, same liquid (or liquid with same viscosity) is adopted in the left and right microfluidic assemblies 201 and 202. The cross-sectional area of the microfluidic channel (e.g., the second microfluidic channel 22) of the right microfluidic assembly 202 is larger than the left microfluidic assembly 201, or the intersection angle 224 of the right microfluidic assembly 202 is larger than the left microfluidic assembly 201. Accordingly, the liquid pressure at right side of the joint 25 is increased, such that the liquid at the joint 25 flows from right toward left (i.e., backward flow). In this situation, as sperms have an instinct for moving upstream, the sperms thus move from left toward right. On the other hand, when the cross-sectional area of the microfluidic channel (e.g., the second microfluidic channel 22) of the right microfluidic assembly 202 is smaller than the left microfluidic assembly 201, or the intersection angle 224 of the right microfluidic assembly 202 is smaller than the left microfluidic assembly 201, the liquid pressure at right side of the joint 25 is decreased, such that the liquid at the joint 25 flows from left toward right (i.e., forward flow). Accordingly, the fluid velocity of forward or backward flow may be fine tuned according to requirement or purpose of the biological detection function of a biochip.

In a third exemplary embodiment, different liquids (or liquids with different viscosity) are adopted in the left and right microfluidic assemblies 201 and 202, respectively, provided that the two microfluidic assemblies 201 and 202 are symmetrical to each other. In this situation, liquid flows at the joint 25 with a flow direction form low-viscosity toward high-viscosity.

In a forth exemplary embodiment, the two microfluidic assemblies 201 and 202 are asymmetrical to each other, and different liquids (or liquids with different viscosity) are adopted in the left and right microfluidic assemblies 201 and 202. With proper design, the liquid at the joint 25 may approximately reach standstill.

Although the biochip 200 of the embodiment described above are made up of two microfluidic assemblies 201 and 202, it is appreciated that the number of the microfluidic assemblies may be greater than two. FIG. 3 shows a top view of a biochip 300 according to another embodiment of the present invention. The biochip 300 is made up of three microfluidic assemblies 301, 302 and 303. Second ends 231 of the third microfluidic channels 23 are connected at the joint 25.

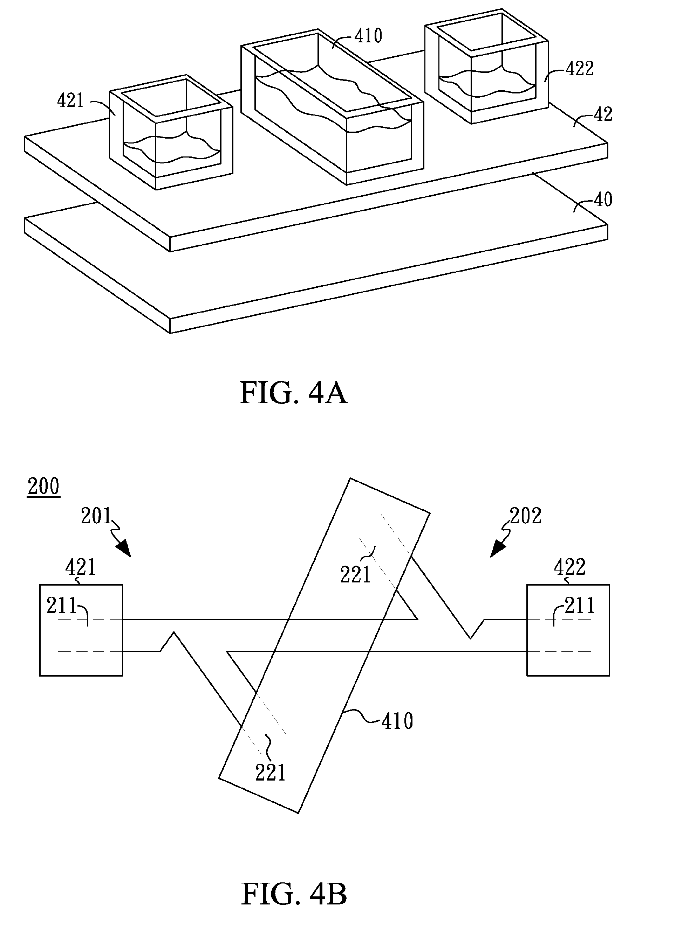

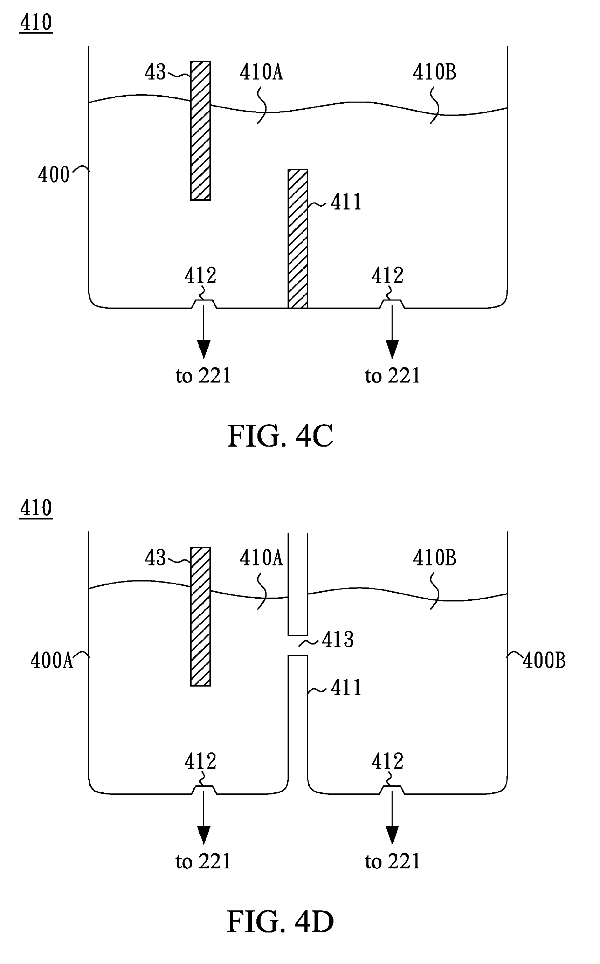

According to one aspect of the embodiment, openings at the second ends 221 of the second microfluidic channels 22 of the microfluidic assemblies (e.g., 201 and 202 in FIG. 2A) are connected to an equal-liquid-level reservoir 410, as shown in a perspective view of FIG. 4A and a top view of FIG. 4B. The equal-liquid-level reservoir 410 is disposed on a cover 42 (e.g., made of polymer). A left waste reservoir 421 (corresponding to the second end 211 of the left first microfluidic channel 21) and a right waste reservoir 422 (corresponding to the second end 211 of the right first microfluidic channel 21) are also disposed on the cover 42. A substrate 40, in which the microfluidic channels (e.g., 21, 22 and 23) are formed, is disposed below the cover 42. FIG. 4C shows a cross-sectional view of the equal-liquid-level reservoir 410 of FIG. 4A.

As exemplified in FIG. 4C, the equal-liquid-level reservoir 410 includes a container 400, which is partitioned into plural (e.g., two in this example) tanks 410A and 410B by a partition wall 411. Openings 412 on a bottom surface of the tanks 410A and 410B communicate with the second ends 221 of the second microfluidic channels 22, respectively. It is noted that the liquid surface of the tanks 410A and 410B is not blocked by the partition wall 411, such that the liquid levels of the tanks 410A and 410B are substantially equal without liquid-level difference therebetween, thereby enhancing accuracy of biological detection and simplifying operation with convenience. FIG. 4D shows another cross-sectional view of the equal-liquid-level reservoir 410 of FIG. 4A. In this example, the equal-liquid-level reservoir 410 includes two containers 400A and 400B, acting as the tanks 410A and 410B, respectively. In lieu of the partition wall 411, a leveling tube 413, below and near the liquid surface, is disposed between the tanks 410A and 410B, such that the liquid levels of the tanks 410A and 410B are substantially equal.

Regarding the equal-liquid-level reservoir 410 of FIG. 4C or FIG. 4D, a blocking wall 43 is disposed in one tank (e.g., 410A). A top of the blocking wall 43 blocks the liquid surface in the tank 410A, and a bottom of the blocking wall 43 does not completely block the liquid in the tank 410A. Accordingly, after a biological specimen (e.g., sperms) pours into the tank 410A, the biological specimen may be contained within the bottom of the tank 410A but not entering into another tank 410B over the partition wall 411 or via the leveling tube 413.

Although specific embodiments have been illustrated and described, it will be appreciated by those skilled in the art that various modifications may be made without departing from the scope of the present invention, which is intended to be limited solely by the appended claims.

## Claims

1. An equal-liquid-level reservoir, adapted to a microfluidic biochip, the equal-liquid-level reservoir comprising:
a plurality of tanks that have a substantially same liquid level, each said tank having an opening on a bottom surface thereof, and each said opening communicating with a corresponding microfluidic channel.

2. The equal-liquid-level reservoir of claim 1, comprising:
a container; and
at least one partition wall disposed in the container to partition the container into the plurality of tanks, in a manner that a liquid surface of the plurality of tanks is not blocked by the partition wall such that liquid levels of the plurality of tanks are substantially equal.

3. The equal-liquid-level reservoir of claim 1, comprising:
a plurality of containers acting as the plurality of tanks respectively; and
a leveling tube disposed below and near a liquid surface of the plurality of tanks such that liquid levels of the plurality of tanks are substantially equal.

4. The equal-liquid-level reservoir of claim 1, further comprising:
a blocking wall disposed in one of the plurality of tanks, a top of the blocking wall blocking a liquid surface in said tank, and a bottom of the blocking wall not completely blocking liquid in said tank.

5. A microfluidic biochip, comprising:
a substrate with microfluidic channels formed therein;
a cover disposed above the substrate; and
an equal-liquid-level reservoir disposed on the cover, the equal-liquid-level reservoir comprising a plurality of tanks that have a substantially same liquid level, each said tank having an opening on a bottom surface thereof, and each said opening communicating with a corresponding microfluidic channel.

6. The microfluidic biochip of claim 5, wherein the equal-liquid-level reservoir comprises:
a container; and
at least one partition wall disposed in the container to partition the container into the plurality of tanks, in a manner that a liquid surface of the plurality of tanks is not blocked by the partition wall such that liquid levels of the plurality of tanks are substantially equal.

7. The microfluidic biochip of claim 5, wherein the equal-liquid-level reservoir comprises:
a plurality of containers acting as the plurality of tanks respectively; and
a leveling tube disposed below and near a liquid surface of the plurality of tanks such that liquid levels of the plurality of tanks are substantially equal.

8. The microfluidic biochip of claim 5, wherein the equal-liquid-level reservoir further comprises:
a blocking wall disposed in one of the plurality of tanks, a top of the blocking wall blocking a liquid surface in said tank, and a bottom of the blocking wall not completely blocking liquid in said tank.

9. The microfluidic biochip of claim 5, wherein the microfluidic channels comprise a plurality of microfluidic assemblies each comprising:
a first microfluidic channel;
a second microfluidic channel;
a third microfluidic channel, first ends of the first microfluidic channel, the second microfluidic channel and the third microfluidic channel being connected at a junction, which has a cross-sectional area smaller than cross-sectional areas of the first microfluidic channel, the second microfluidic channel and the third microfluidic channel; and
a pair of electrodes which are disposed at second ends of the first microfluidic channel and the third microfluidic channel, respectively;
wherein the second end of the third microfluidic channel of one microfluidic assembly is connected at a joint to another microfluidic assembly, and second ends of the second microfluidic channels of the microfluidic assemblies communicate with the openings of the tanks.

10. The microfluidic biochip of claim 9, wherein the first microfluidic channel and the third microfluidic channel are arranged in a substantially straight line, and an intersection angle is defined between the second microfluidic channel and the third microfluidic channel, such that fluid velocity from the second microfluidic channel toward the first microfluidic channel is different from fluid velocity from the second microfluidic channel toward the third microfluidic channel.

11. The microfluidic biochip of claim 9, wherein the intersection angle of one microfluidic assembly is greater than other microfluidic assembly, such that liquid at the joint flows from the microfluidic assembly with larger intersection angle toward other microfluidic assembly.

12. The microfluidic biochip of claim 9, wherein cross-sectional area of the second microfluidic channel of one microfluidic assembly is greater than other microfluidic assembly, such that liquid at the joint flows from the microfluidic assembly with larger cross-sectional area of the second microfluidic channel toward other microfluidic assembly.

13. The microfluidic biochip of claim 9, wherein liquid viscosity of one microfluidic assembly is higher than other microfluidic assembly, such that liquid at the joint flows from the microfluidic assembly with higher viscosity toward other microfluidic assembly.

14. The microfluidic biochip of claim 9, wherein the electrode disposed at the second end of the third microfluidic channel is a common electrode among the microfluidic assemblies.

15. The microfluidic biochip of claim 9, further comprising at least one waste reservoir disposed on the cover and connected to the second end of the first microfluidic channel.
